(19) Europäisches Patentamt
European Patent Office
Office européen des brevets

(11) Veröffentlichungsnummer: **0 529 212 B1**

(12) **EUROPÄISCHE PATENTSCHRIFT**

(45) Veröffentlichungstag der Patentschrift: **30.08.95**

(51) Int. Cl.6: **A23G 3/30**, A61K 7/16

(21) Anmeldenummer: **92109443.9**

(22) Anmeldetag: **04.06.92**

(54) **Kaugummimasse, Verfahren zu ihrer Herstellung und Verwendung.**

(30) Priorität: **16.07.91 DE 4123450**

(43) Veröffentlichungstag der Anmeldung:
**03.03.93 Patentblatt 93/09**

(45) Bekanntmachung des Hinweises auf die
Patenterteilung:
**30.08.95 Patentblatt 95/35**

(84) Benannte Vertragsstaaten:
**AT BE CH DE DK ES FR GB GR IT LI LU MC
NL PT SE**

(56) Entgegenhaltungen:
**EP-A- 0 091 611
WO-A-85/04098
WO-A-89/08989
CH-A- 646 843**

**PATENT ABSTRACTS OF JAPAN Band 199,
Nr. 8 (C-242), 12. September 1984**

(73) Patentinhaber: **STAFFORD-MILLER CONTINEN-
TAL, NV-SA
Nijverheidsstraat 9
B-2131 Oevel (BE)**

Patentinhaber: **BLOCK DRUG COMPANY INC.
Am Rosenkothen 4
D-40880 Ratingen (DE)**

(72) Erfinder: **Hoburg, Albrecht, Dr.
Schumannsdieken 16
W-4030 Ratingen 4 (DE)**
Erfinder: **Schäfers, Wolfgang
Schwetzinger Strasse 21
W-5000 Köln 91 (DE)**

(74) Vertreter: **Werner, Hans-Karsten, Dr. et al
Patentanwälte
Von Kreisler-Selting-Werner
Postfach 10 22 41
D-50462 Köln (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist eine Kaugummimasse sowie ein Verfahren zu ihrer Herstellung und eine Verwendung.

Es ist bekannt, in Zahnpflegemitteln, wie Zahncremes, Mundwässern, Tabletten und Mundgelen Fluorverbindungen zur Kariesprophylaxe einzusetzen.

In einigen europäischen Ländern wird aus diesem Grunde auch schon das Trinkwasser entsprechend fluoriert. Weiterhin ist für die Aufrechterhaltung eines gesunden widerstandsfähigen Zahnschmelzes eine ausreichende Zufuhr von Calcium-Ionen erforderlich. Diese sind daher in Zahnpflegemitteln im allgemeinen in Form von Calcium oder Natriumphosphaten enthalten.

Eine wirksame Kariesprophylaxe ist jedoch nur bei regelmäßiger Reinigung der Zähne mit Zahnpflegemitteln, insbesondere nach den Mahlzeiten möglich. Obwohl die Notwendigkeit der regelmäßigen Zahnreinigung allgemein bekannt ist, ist Karies inzwischen zu einer weit verbreiteten Krankheit geworden, an der Menschen jeden Alters erkranken.

Dies zeigt, daß die Zahnpflege häufig vernachlässigt wird. Insbesondere während des Tages werden Mahlzeiten eingenommen, ohne daß die Zähne anschließend gereinigt werden. Für Berufstätige scheint es zu umständlich zu sein, ständig Zahnbürste und Zahncreme mitzuführen. Es bedarf daher während des Tages einer einfachen, leicht transportablen Ergänzung der Zahnpflege.

Es ist bekannt, daß Kaugummi zur Zahnpflege verwendet werden kann. Solche Kaugummimassen, wie sie in der DE-32 13 284 beschrieben werden, enthalten neben üblichen Bestandteilen einer Kaugummimasse Fluoride und Calciumphosphat als zusätzliche Bestandteile mit zahnpflegenden Eigenschaften. Als Zuckeraustauschstoff wurde bisher in Kaugummimassen im allgemeinen Sorbit verwendet.

Aus der WO 85/04098 ist weiterhin bekannt, daß lösliche Kaliumsalze in der Kaugummimasse vorteilhaft auf hypersensible Zähne wirken.

Die vorliegende Erfindung stellt nun eine Verbesserung einer solchen Kaugummimasse zur Zahnpflege zur Verfügung. Es wurde nämlich gefunden, daß der Zuckeraustauschstoff Xylit, der aus Baumrinde gewonnen wird, in Kaugummi gegen Karies wirksam ist, die Zahnbelagbildung verringert und die Remineralisierung des Zahnschmelzes unterstützt.

Das technische Problem der Erfindung war daher die Weiterentwicklung der bisher bekannten Kaugummimassen zur Zahnpflege.

Das technische Problem wird wie im Anspruch 1 beschrieben gelöst.

Die Kaugummimasse enthält 0,01 bis 5 Gew.-% eines wasserlöslichen Kaliumsalzes. Besonders bevorzugt sind Kaliumchlorid und Kaliumhydrogencarbonat. Weiterhin ist Phosphat, vorzugsweise Tricalciumphosphat und/oder Natriumphosphat, in einer Menge von 1 bis 5 Gew.-% enthalten. Als Zuckeraustauschstoff wird Xylit oder Xylit und Sorbit in einer Menge von mindestens 50 Gew.-% verwendet.

Weiterhin sind für Kaugummimassen übliche Inhaltsstoffe enthalten. Insbesondere kann auch Fluorid beigemischt werden.

In einer bevorzugten Ausführungsform enthält die erfindungsgemäße Kaugummimasse 1 bis 10 Gew.-% Strontiumchlorid und/oder Strontiumacetat.

Die erfindungsgemäßen Kaugummimassen werden durch Mischen der Einzelkomponenten in einem üblichen Mischer hergestellt. Sie sind insbesondere geeignet zur Verwendung als Zahnpflegemittel.

Im folgenden wird die Erfindung anhand eines Ausführungsbeispiels näher beschrieben:

Ausführungsbeispiel:

Durch übliches Mischen und anschließendes Homogenisieren wird eine Kaugummimasse mit folgender Zusammensetzung hergestellt (Angaben in Gew.-%):

| | |
|---|---|
| Kaumasse | 38,97 |
| Zuckeraustauschstoffe | |
| Sorbit | 26,18 |
| Xylit | 26,58 |
| Süßstoff | 0,74 |
| Aromen | 1,48 |
| Verdickungsmittel | 0,92 |
| Oberflächenbehandlungsmittel | 0,09 |
| Wirkstoffe | |
| Kaliumchlorid | 0,85 |
| Natrium-Monofluor-Phosphat | 0,04 |
| Tricalciumphosphat | 2,98 |

**Patentansprüche**

1. Kaugummimasse enthaltend 0,01 bis 5 Gew.-% eines wasserlöslichen Kaliumsalzes, 1 bis 5 Gew.-% eines Phosphates und mindestens 50 Gew.-% Xylit oder Xylit und Sorbit neben für Kaugummimassen üblichen Inhaltsstoffen.

2. Kaugummimasse nach Anspruch 1, dadurch gekennzeichnet, daß als Kaliumsalz Kaliumchlorid und/oder Kaliumhydrogencarbonat enthalten sind.

3. Kaugummimasse nach Anspruch 1 oder 2, dadurch gekennzeichnet, daß Tricalciumphosphat und/oder Natriumphosphat enthalten sind.

4. Kaugummimasse nach den Ansprüchen 1 bis 3, dadurch gekennzeichnet, daß zusätzlich eine Fluor enthaltende Verbindung enthalten ist.

5. Kaugummimasse nach den Ansprüchen 1 bis 4, dadurch gekennzeichnet, daß zusätzlich 1 bis 10 Gew.-% Strontiumchlorid und/oder Strontiumacetat enthalten sind.

6. Kaugummimasse nach den Ansprüchen 1 bis 4, dadurch gekennzeichnet, daß statt eines wasserlöslichen Kaliumsalzes Strontiumchlorid und/oder Strontiumacetat enthalten sind.

7. Verfahren zur Herstellung der Kaugummimasse, dadurch gekennzeichnet, daß 0,01 bis 5 Gew.-% wasserlösliches Kaliumsalz mit 1 bis 5 Gew.-% eines Phosphates und mindestens 50 Gew.-% Xylit oder Xylit und Sorbit mit für Kaugummimassen üblichen Inhaltsstoffen vermischt werden.

**Claims**

1. A chewing gum mass, containing from 0.01 to 5% by weight of a water-soluble potassium salt, from 1 to 5% by weight of a phosphate, and at least 50% by weight of xylitol or xylitol and sorbitol in addition to usual ingredients for chewing gum masses.

2. The chewing gum mass according to claim 1, characterized in that potassium chloride and/or potassium hydrogen-carbonate are contained as the potassium salt.

3. The chewing gum mass according to claims 1 or 2, characterized in that tricalcium phosphate and/or sodium phosphate are contained.

**4.** The chewing gum mass according to claims 1 to 3, characterized in that a fluorine-containing compound is additionally contained.

**5.** The chewing gum mass according to claims 1 to 4, characterized in that from 1 to 10% by weight of strontium chloride and/or strontium acetate are additionally contained.

**6.** The chewing gum mass according to claims 1 to 4, characterized in that strontium chloride and/or strontium acetate are contained instead of a water-soluble potassium salt.

**7.** A process for producing said chewing gum mass, characterized in that from 0.01 to 5% by weight of a water-soluble potassium salt is mixed with from 1 to 5% by weight of a phosphate and at least 50% by weight of xylitol or xylitol and sorbitol and usual ingredients for chewing gum masses.

**Revendications**

**1.** Chewing-gum contenant 0,01 à 5 % en poids de sel de potassium soluble dans l'eau, 1 à 5 % en poids d'un phosphate et au moins 50 % en poids de xylite ou de xylite et sorbite à côté des ingrédients habituels du chewing-gum.

**2.** Chewing-gum selon la revendication 1, caractérisé en ce qu'il contient, comme sel de potassium, le chlorure de potassium et/ou le carbonate de potassium et hydrogène.

**3.** Chewing-gum selon la revendication 1 ou 2, caractérisé en ce qu'il contient du phosphate tri-calcique ou du phosphate de sodium.

**4.** Chewing-gum selon les revendications 1 à 3, caractérisé en ce qu'il contient en outre un composé contenant du fluor.

**5.** Chewing-gum selon les revendications 1 à 4, caractérisé en ce qu'il contient en outre 1 à 10 % en poids de chlorure et/ou d'acétate de strontium.

**6.** Chewing-gum selon les revendications 1 à 4, caractérisé en ce qu'au lieu d'un sel de potassium soluble dans l'eau, il contient du chlorure et/ou de l'acétate de strontium.

**7.** Procédé d'obtention de chewing-gum, caractérisé en ce qu'on mélange 0,01 à 5 % en poids de sel de potassium soluble dans l'eau avec 1 à 5 % en poids d'un phosphate et au moins 50 % en poids de xylite ou xylite et sorbite avec les ingrédients habituels du chewing-gum.